# EUROPEAN PATENT SPECIFICATION

(11) **EP 3 832 296 B1**
(45) Date of publication and mention of the grant of the patent: **28.12.2022**
(21) Application number: 19840187.9
(22) Date of filing: 25.04.2019
(51) Int. Cl.: G01N 27/327, G01N 27/31

(54) **METHOD FOR PREPARING BIOSENSING FILM, BIOSENSING FILM AND MONITORING DEVICE**
VERFAHREN ZUR HERSTELLUNG EINES BIOSENSORFILMS, BIOSENSORFILM UND ÜBERWACHUNGSVORRICHTUNG
MÉTHODE DE PRÉPARATION D'UN FILM DE BIODÉTECTION, FILM DE BIODÉTECTION ET DISPOSITIF DE SURVEILLANCE

(30) Priority: 27.07.2018 CN 201810846227
(43) Date of publication of application: 09.06.2021
(73) Proprietor: Sinocare Inc., Changsha, Hunan 410205 (CN)
(72) Inventor: GAO, Zhiqiang, Changsha, Hunan 410205 (CN)
(74) Representative: Monteiro Alves, Inês
(86) International application number: PCT/CN2019/084254
(87) International publication number: WO 2020/019786

(56) References cited:
- EP-A2- 0 757 246
- WO-A1-92/12254
- WO-A1-2011/037702
- CN-A- 1 776 414
- CN-A- 107 037 101
- CN-A- 108 918 625
- US-A- 5 264 104
- US-A1- 2015 232 913

## Description

### TECHNICAL FIELD

This disclosure generally relates to the technical field of detection devices, and the invention relates to a method for preparing a biosensing membrane.

### BACKGROUND

An electrochemical biosensor is a device sensitive to a biological substance and capable of converting its concentration to an electrical signal for detection. It comprises a selective biological substance such as an oxidoreductase or an antibody capable of identifying a target substance, as well as an electrode and an auxiliary device thereof for converting the identified signal into an electrical signal. For instance, when the oxidoreductase is used as the target-identifying substance, the electron exchange between the oxidoreductase and the electrode is an important step performed by a biosensor. Normally, the redox active sites of the oxidoreductase do not exchange electrons with the electrode directly, and thus the electron transfer between the redox active sites and the electrode is a limiting factor for most biosensors. To solve the technical problem relating to the low efficiency of electron transfer, a redox active molecule with excellent electrochemical properties may be introduced into the biosensing membrane to establish an electron transfer channel between the oxidoreductase and the electrode such that a quick electron exchange is achieved.

US 5,264,104 discloses a membrane for a biosensor having good electron transfer properties.

However, after the introduction of the redox active molecule, how to prepare a stable biosensing membrane using the redox active molecule and the oxidoreductase, how to fix the membrane on the electrode, and how to stably perform a plurality of detections, especially perform detections while ensuring the stability of detection results after being implanted in a living body are technical challenges that need to be solved urgently for those skilled in the art.

### SUMMARY

The purpose of the present invention is to provide a method for preparing a biosensing membrane. . The biosensing membrane formed by the aforesaid preparation method is stable and durable, may be used to perform a plurality of detections, and is particularly suitable to serve as a biosensing membrane of a living body monitoring device.

To achieve the above purpose, the present invention is a method as defined by claim 1. The invention adopts the following technical solution: a method for preparing a biosensing membrane, comprising the steps of: electrochemically activating and modifying an oxidoreductase, then performing a cross-linking treatment using a chemical cross-linking agent, and then coating on a surface of an electrode, thereby forming a biosensing membrane, wherein the chemical cross-linking agent is glutaraldehyde or polyethylene glycol diglycidyl ether.

In another preferred embodiment, the oxidoreductase is one or more substances selected from glucose oxidase, glucose dehydrogenase, lactate oxidase, lactate dehydrogenase and catalase.

The oxidoreductase is electrochemically activated and modified using a ruthenium complex having free amino or carboxyl groups or an osmium complex having free amino or carboxyl groups.

In another preferred embodiment, electrochemically activating and modifying the oxidoreductase, comprising the steps of: uniformly mixing the oxidoreductase with the ruthenium complex having free amino or carboxyl groups or the osmium complex having free amino groups in a buffer solution, sequentially adding carbodiimide and N-hydroxysuccinimide, uniformly mixing, reacting at a temperature of 2-6°C for 12-48 hours, and performing the dialysis using a dialysis bag.

In another preferred embodiment, the oxidoreductase is modified twice using the ruthenium complex having free amino or carboxyl groups or the osmium complex having free amino groups or carboxyl groups, wherein the molecular weight cut off in the preliminary modification is 5000 to 50000, and the molecular weight cut off in the secondary modification is 500 to 50000.

In another preferred embodiment, the osmium complex having free amino or carboxyl groups is Os(bpy)₂(3-aminopropyl imidazole)Cl or Os(bpy)₂(4-imidazole butyric acid)Cl, and the ruthenium complex having free amino or carboxyl groups is Ru(bpy)₂(3-aminopropyl imidazole)Cl or Os(bpy)₂(4-imidazole butyric acid)Cl.

In another preferred embodiment, performing a cross-linking treatment using a chemical cross-linking agent, comprising the steps of: uniformly mixing the modified oxidoreductase with the chemical cross-linking agent in a buffer solution, reacting for 0.5-5 hours and coating on the surface of the electrode, thereby forming a biosensing membrane.

In another preferred embodiment, after the biosensing membrane formed by the cross-linking treatment is dried, a polyvinyl pyridine and Nafion mixed alcoholic solution are coated on the surface of the biosensing membrane, thereby forming a biosensing membrane with biocompatibility.

A biosensing membrane prepared by any one of the aforesaid preparation methods is disclosed but not forming part of the invention. .

A monitoring device comprising a sensor, wherein the sensor comprises a biosensing membrane prepared by any one of the aforesaid preparation methods is disclosed but not forming part of the invention.

The present invention provides a method for preparing a biosensing membrane:
electrochemically activating and modifying an oxidoreductase, then performing a cross-linking treatment using a chemical cross-linking agent, and then coating on a surface of an electrode, thereby forming a biosensing membrane, wherein the chemical cross-linking agent is glutaraldehyde or polyethylene glycol diglycidyl ether. According to the preparation method of the present disclosure, glutaraldehyde or polyethylene glycol diglycidyl ether is used as the chemical cross-linking agent. The modified oxidoreductase undergoes a cross-linking treatment and is then coated on a surface of an electrode, thereby forming a biosensing membrane on the surface of the electrode. The biosensing membrane formed by means of the cross-linking treatment using glutaraldehyde or polyethylene glycol diglycidyl ether is stable and durable, may be used to perform a plurality of detections, and is particularly suitable to serve as a biosensing membrane of a living body monitoring device.

### BRIEF DESCRIPTION OF THE DRAWINGS

Apparently, the figures in the following description are merely some embodiments recorded in the present disclosure. For those skilled in the art, other figures may be obtained based on the aforesaid figures without paying creative effort.

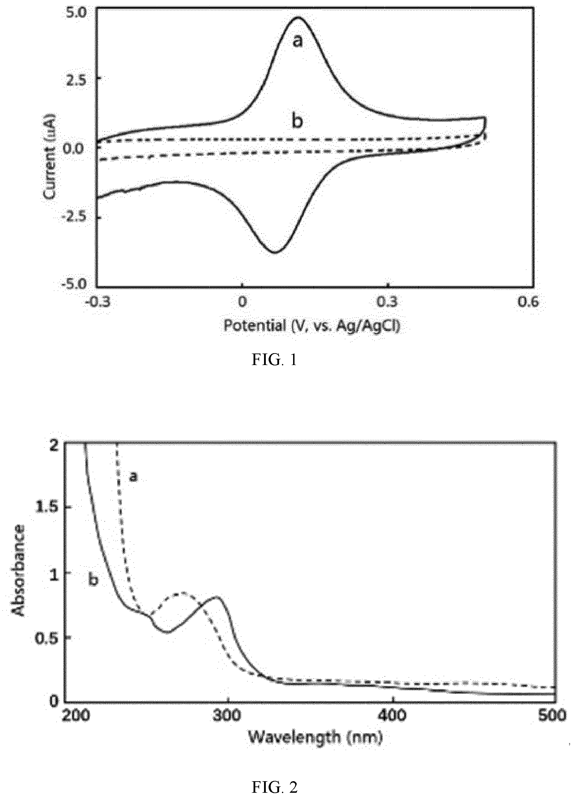

Figure 1 is a cyclic voltammogram shows a comparison between the glucose oxidase modified using Os(bpy)₂(3-aminopropyl imidazole)Cl and the natural glucose oxidase, wherein (a) is a cyclic voltammogram of the glucose oxidase modified using Os(bpy)₂(3-aminopropyl imidazole)Cl and (b) is a cyclic voltammogram of the natural glucose oxidase.
Figure 2 is a UV-visible absorption spectrum shows a comparison between the glucose oxidase modified using Os(bpy)₂(3-aminopropyl imidazole)Cl and the natural glucose oxidase, wherein (a) is a UV-visible absorption spectrum of the glucose oxidase modified using Os(bpy)₂(3-aminopropyl imidazole)Cl and (b) is a UV-visible absorption spectrum of the natural glucose oxidase.

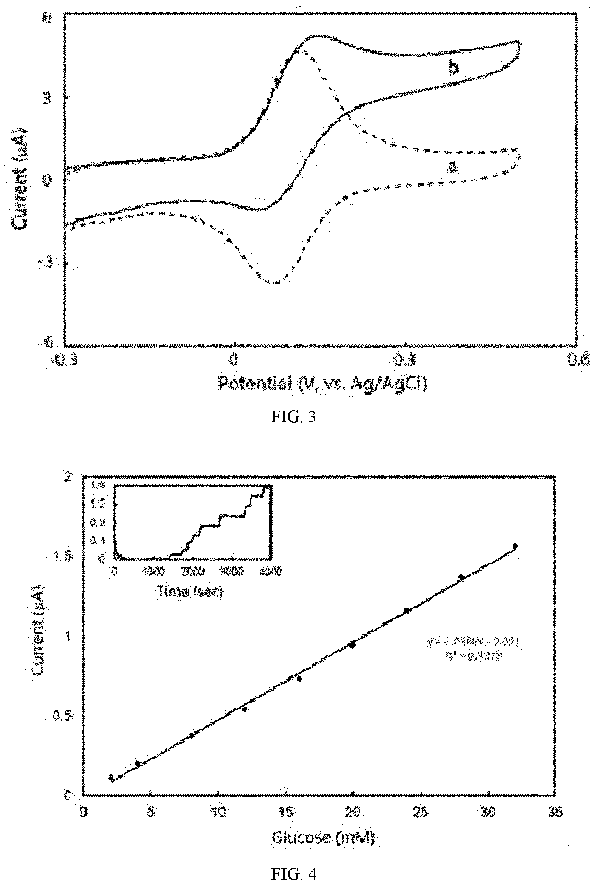

Figure 3 is a cyclic voltammogram shows a comparison between the biosensing membrane containing the modified glucose oxidase of the present disclosure in a pH 7.4 phosphate-buffered saline (PBS) buffer solution and after adding 5.0 mM glucose, wherein (a) is a cyclic voltammogram of the biosensing membrane containing the modified glucose oxidase in a PBS (pH 7.4) buffer solution and (b) is a cyclic voltammogram after adding 5.0 mM glucose.
Figure 4 is a conceptual diagram showing the relationship between the electrochemical catalytic oxidation current of glucose on the biosensing membrane and the glucose concentration in the embodiment of the present disclosure.

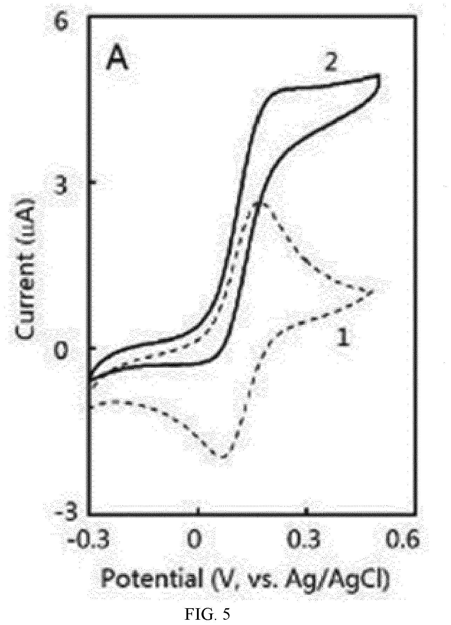

Figure 5 is a cyclic voltammogram shows a comparison between the biosensing membrane containing the modified lactate oxidase of the present disclosure in a PBS (pH 7.4) buffer solution and after adding 5.0 mM lactate, wherein (1) is a cyclic voltammogram of the biosensing membrane containing the modified lactate oxidase in a PBS (pH 7.4) buffer solution and (2) is a cyclic voltammogram after adding 5.0 mM lactate.

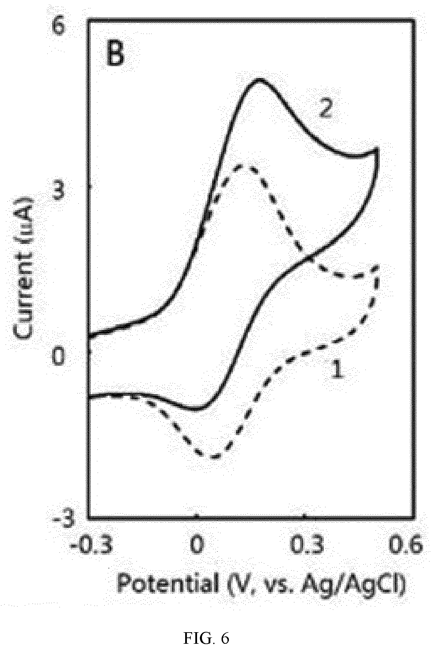

Figure 6 is a cyclic voltammogram shows a comparison between the biosensing membrane containing the modified glucose dehydrogenase of the present disclosure in a PBS (pH 7.4) buffer solution and after adding 5.0 mM glucose, wherein (1) is a cyclic voltammogram of the biosensing membrane containing the modified glucose dehydrogenase in a PBS (pH 7.4) buffer solution and (2) is a cyclic voltammogram after adding 5.0 mM glucose.

### DETAILED DESCRIPTION

To allow those skilled in the art to better understand the technical solution of the present invention, figures are combined hereinafter to clearly and completely describe the technical solution of the present invention.

Obviously, the described embodiments are merely a part but not all of the embodiments of the present invention. Other embodiments falling into the scope of claim 1 should be part of the invention.

As shown in Figures 1-6, the present invention provides a method for preparing a biosensing membrane: modifying an oxidoreductase, then performing a cross-linking treatment using a chemical cross-linking agent, and then coating on a surface of an electrode, thereby forming a biosensing membrane, wherein the chemical cross-linking agent is glutaraldehyde or polyethylene glycol diglycidyl ether.

The present invention comprises electrochemically activating and modifying an oxidoreductase, then performing a cross-linking treatment using a chemical cross-linking agent, and then coating on a surface of an electrode, thereby forming a biosensing membrane, wherein the chemical cross-linking agent is glutaraldehyde or polyethylene glycol diglycidyl ether. According to the preparation method of the present disclosure, glutaraldehyde or polyethylene glycol diglycidyl ether is used as the chemical cross-linking agent. The modified oxidoreductase undergoes a cross-linking treatment and is then coated on a surface of an electrode, thereby forming a biosensing membrane on the surface of the electrode. The biosensing membrane formed by means of the cross-linking treatment using glutaraldehyde or polyethylene glycol diglycidyl ether is stable and durable, may be used to perform a plurality of detections, and is particularly suitable to serve as a biosensing membrane of a living body monitoring device.

Preferably, the oxidoreductase is one or more substances selected from glucose oxidase, glucose dehydrogenase, lactate oxidase, lactate dehydrogenase and catalase.

In the present disclosure, more specifically, the oxidoreductase is one or more substances selected from glucose oxidase, glucose dehydrogenase, lactate oxidase, lactate dehydrogenase and catalase. After being electrochemically activated and modified, and then treated using a chemical cross-linking agent, a stable biosensing membrane is formed on the surface of the electrode for detecting a target substance.

The oxidoreductase is electrochemically activated and modified using a ruthenium complex having free amino or carboxyl groups or an osmium complex having free amino or carboxyl groups.

Preferably, modifying the oxidoreductase comprising the steps of: uniformly mixing the oxidoreductase with the ruthenium complex having free amino or carboxyl groups or the osmium complex having free amino or carboxyl groups in a buffer solution, sequentially adding carbodiimide and N-hydroxysuccinimide, uniformly mixing, reacting at a temperature of 2-6°C for 12-48 hours, and performing the dialysis using a dialysis bag.

Preferably, the oxidoreductase is modified for twice using the ruthenium complex having free amino or carboxyl groups or the osmium complex having free amino or carboxyl groups, wherein the molecular weight cut off in the preliminary modification is 5000 to 50000, and the molecular weight cut off in the secondary modification is 500 to 50000.

Preferably, the osmium complex having free amino or carboxyl groups is Os(bpy)₂(3-aminopropyl imidazole)Cl or Os(bpy)₂(4-imidazole butyric acid)Cl, and the ruthenium complex having free amino or carboxyl groups is Ru(bpy)₂(3-aminopropyl imidazole)Cl or Os(bpy)₂(4-imidazole butyric acid)Cl.

In the present disclosure, preferably, the oxidoreductase is modified using the ruthenium complex having free amino or carboxyl groups or the osmium complex having free amino or carboxyl groups, and more preferably, the oxidoreductase is modified using Os(bpy)₂(3-aminopropyl imidazole)Cl or Ru(bpy)₂(3-aminopropyl imidazole)Cl, wherein bpy refers to 2,2-bipyridine.

Electrochemically activating and modifying the oxidoreductase, comprising the steps of: uniformly mixing the oxidoreductase with the ruthenium complex having free amino groups or the osmium complex having free amino groups in a buffer solution, sequentially adding carbodiimide and N-hydroxysuccinimide, uniformly mixing, reacting at a temperature of 2-6°C for 12-48 hours, and performing the dialysis using a dialysis bag. More preferably, the oxidoreductase is modified for twice using the ruthenium complex having free carboxyl groups or the osmium complex having free carboxyl groups, wherein the molecular weight cut off in the preliminary modification is 5000 to 50000, and the molecular weight cut off in the secondary modification is 500 to 50000.

More specifically, electrochemically activating and modifying the oxidoreductase for twice using the ruthenium complex having free amino or carboxyl groups or the osmium complex having free amino or carboxyl groups, comprising the steps of: uniformly mixing the oxidoreductase (e.g., glucose oxidase) with the ruthenium complex having free amino groups or the osmium complex having free amino groups in a PBS buffer solution, sequentially adding carbodiimide and N-hydroxysuccinimide, uniformly mixing, reacting at a temperature of 2-6°C for 12-48 hours, and performing the dialysis using a dialysis bag, wherein the molecular weight cut off is 5000 to 50000; subsequently, separating and purifying the preliminarily modified oxidoreductase, adding the ruthenium complex having free carboxyl groups or the osmium complex having free carboxyl groups into the purified oxidoreductase solution, sequentially adding carbodiimide and N-hydroxysuccinimide, uniformly mixing, and reacting at a temperature of 2-6°C for 12-48 hours; after the reaction is completed, re-performing the dialysis using a dialysis bag, wherein the molecular weight cut off is 500 to 50000; finally, separating and purifying the secondarily modified oxidoreductase, wherein the concentration of the ruthenium complex having free amino or carboxyl groups or the osmium complex having free amino or carboxyl groups is preferably 0.1-50 mg/ml and more preferably 1-20 mg/ml, the concentration of the carbodiimide is preferably 0.1-50 mmol/L and more preferably 0.1-25 mmol/L, and the concentration of the N-hydroxysuccinimide is preferably 0.01-5 mmol/L. The spectrophotometric analysis proves that the oxidoreductase has been successfully modified.

Preferably, performing a cross-linking treatment using a chemical cross-linking agent, comprising the steps of: uniformly mixing the modified oxidoreductase with the chemical cross-linking agent in a buffer solution, reacting for 0.5-5 hours and coating on the surface of the electrode, thereby forming a biosensing membrane.

Preferably, after the biosensing membrane formed by the cross-linking treatment is dried, a polyvinyl pyridine and a Nafion mixed alcoholic solution are coated on the surface of the biosensing membrane, thereby forming a biosensing membrane with biocompatibility.

In the present disclosure, more specifically, performing a cross-linking treatment using a chemical cross-linking agent, comprising the steps of: uniformly mixing the modified oxidoreductase with the chemical cross-linking agent in a buffer solution, reacting for 0.5-5 hours and coating on the surface of the electrode, thereby forming a biosensing membrane; preferably, after the biosensing membrane formed by the cross-linking treatment is dried, coating a polyvinyl pyridine and a Nafion mixed alcoholic solution on the surface of the biosensing membrane, thereby forming a biosensing membrane with biocompatibility.

More specifically, making the modified oxidoreductase react with a chemical cross-linking agent, comprising the steps of: fully mixing the modified oxidoreductase with a glutaraldehyde solution or a polyethylene glycol diglycidyl ether solution in a PBS buffer solution, reacting for 0.5-5 hours, preferably 0.5-3 hours, and coating the chemically crosslinked oxidoreductase on the surface of the electrode through adopting a drop-casting method or a dip-coating method, thereby forming a biosensing membrane; after the biosensing membrane on the electrode is completely dried, coating a polyvinyl pyridine and a nafion mixed alcoholic solution on the surface of the biosensing membrane through adopting a drop-casting method or a dip-coating method, thereby forming a biosensing membrane with biocompatibility. Preferably, the concentration of the chemical cross-linking agent (glutaraldehyde solution or polyethylene glycol diglycidyl ether solution) is 0.1-5%, the concentration of the modified oxidoreductase is preferably 5-150 mg/ml, the concentration of the polyvinyl pyridine solution is preferably 20-300 mg/ml, and the concentration of the nafion mixed alcoholic solution is preferably 0.1-5%. Preferably, a mixed ethanol solution containing polyvinyl pyridine and Nafion is adopted, namely, polyvinyl pyridine and Nafion being dissolved and mixed in alcohol to obtain a mixed ethanol solution containing polyvinyl pyridine and Nafion.

After performing the cross-linking treatment, the oxidoreductase maintains its direct electrochemical action. Experiments show that the modified glucose oxidase maintains its performance of catalytic oxidation for glucose in the biosensing membrane, and the catalytic oxidation efficiency of the modified glucose oxidase for glucose through direct electrochemistry is 140 times higher than that of the natural glucose oxidase for glucose through oxygen.

A biosensing membrane, not forming part of the invention, is prepared by any one of the aforesaid preparation methods.

A monitoring device, not forming part of the invention, comprises a sensor, wherein the sensor comprises a biosensing membrane prepared by any one of the aforesaid preparation methods.

The present disclosure provides a biosensing membrane prepared by any one of the aforesaid preparation methods and a monitoring device comprising a sensor, wherein the sensor comprises a biosensing membrane prepared by any one of the aforesaid preparation methods.

Preferably, the monitoring device is an implantable continuous monitoring device, which may stably operate after being implanted in a life body (such as a human body), and may perform a plurality of detections for a target substance for a long time. According to the implantable continuous monitoring device, a sensor fixed with a biosensing membrane is implanted under the skin, and a receiver or a mobile device receives the data from the sensor in real time, thereby realizing a long-term continuous monitoring of the concentration of the target substance. For instance, an implantable blood glucose monitoring device may be developed and used for monitoring the blood glucose and supplementing insulin in time using an insulin pump, thereby regulating the blood glucose contained in the human body.

### Embodiment 1

In this embodiment, modifying the oxidoreductase, comprising the steps of: sufficiently mixing the glucose oxidase with 1-20 mg/ml Os(bpy)₂(3-aminopropyl imidazole)Cl in a PBS buffer solution, sequentially adding 0.1-25 mmol/L carbodiimide and 0.01-5 mmol/L N-hydroxysuccinimide, sufficiently mixing, reacting at a temperature of 4°C for 24 hours, and performing the dialysis using a dialysis bag, wherein the molecular weight cut off is 5000 to 50000; subsequently, separating and purifying the preliminarily modified oxidoreductase, adding 1-20 mg/ml Os(bpy)₂(4-imidazole butyric acid)Cl into the purified oxidoreductase solution, sequentially adding 0.1-25 mmol/L carbodiimide and 0.01-5 mmol/L N-hydroxysuccinimide, sufficiently mixing, and reacting at a temperature of 4°C for 24 hours; after the reaction is completed, re-performing the dialysis using a dialysis bag, wherein the molecular weight cut off is 500 to 50000; finally, separating and purifying the secondarily modified oxidoreductase. After the glucose oxidase is modified, its catalytic active center may directly quickly exchange electrons with the electrode (shown in Figure 1). The spectrophotometric analysis proves that the oxidoreductase has been successfully modified (shown in Figure 2).

Making the modified oxidoreductase react with a chemical cross-linking agent, comprising the steps of: fully mixing the modified 5-150 mg/ml oxidoreductase with a 0.1-5% glutaraldehyde solution in a PBS buffer solution, reacting for 0.5-3 hours, and coating the chemically crosslinked oxidoreductase on the surface of the electrode through adopting a drop-casting method or a dip-coating method, thereby forming a biosensing membrane; after the biosensing membrane on the electrode is completely dried, coating a mixed ethanol solution containing 20-300 mg/ml polyvinyl pyridine and 0.1-5% Nafion on the surface of the biosensing membrane through adopting a drop-casting method or a dip-coating method, thereby forming a biosensing membrane with biocompatibility. After performing the cross-linking treatment using a glutaraldehyde solution, the oxidoreductase maintains its direct electrochemical action, and the prepared biosensing membrane shows an ideal electrochemical performance (shown in Figure 3(a)). After adding 5.0mM glucose into the PBS buffer solution, the cyclic voltammogram of the biosensing membrane clearly shows a typical electrochemical catalytic process (shown in Figure 3(b)). Experiments show that the modified glucose oxidase maintains its performance of catalytic oxidation for glucose in the biosensing membrane, and the catalytic oxidation efficiency of the modified glucose oxidase for glucose through direct electrochemistry is 140 times higher than that of the natural glucose oxidase for glucose through oxygen.

Based on the aforesaid, the biosensing membrane is adopted in an implantable glucose continuous monitoring system. The preliminary experimental result shows that the operating curve is in an ideal linear state between 2.0-32 mM (shown in Figure 4). It is an implantable glucose continuous monitoring system having the widest linear range.

In addition to glucose oxidase, other oxidoreductases such as lactate oxidase and glucose dehydrogenase may also be successfully modified and chemically crosslinked with glutaraldehyde.

Experiments show that the biosensing membranes containing modified lactate oxidase and glucose dehydrogenase show good electrochemical performance on the electrode (shown in Figure 5 and 6). After adding 5.0 mM lactate or glucose into the PBS buffer solution, the cyclic voltammogram of the biosensing membranes clearly show a typical electrochemical catalytic process (shown in Figure 5 and 6). These results indicate that they maintain their catalytic oxidation performance through direct electrochemistry in the biosensing membranes.

The aforesaid description of the disclosed embodiment enables those skilled in the art to realize or use the present invention.

## Claims

1. A method for preparing a biosensing membrane, **characterized by** comprising the steps of: electrochemically activating and modifying an oxidoreductase using a ruthenium complex having free amino or carboxyl groups or an osmium complex having free amino or carboxyl groups, then performing a cross-linking treatment using a chemical cross-linking agent, and then coating on a surface of an electrode, thereby forming a biosensing membrane, wherein the chemical cross-linking agent is glutaraldehyde or polyethylene glycol diglycidyl ether.

2. The preparation method of claim 1, wherein the oxidoreductase is one or more substances selected from glucose oxidase, glucose dehydrogenase, lactate oxidase, lactate dehydrogenase and catalase.

3. The preparation method of claim 1, wherein electrochemically activating and modifying the oxidoreductase, comprising the steps of: uniformly mixing the oxidoreductase with the ruthenium complex having free amino or carboxyl groups or the osmium complex having free amino groups in a buffer solution, sequentially adding carbodiimide and N-hydroxysuccinimide, uniformly mixing, reacting at a temperature of 2-6°C for 12-48 hours, and performing the dialysis using a dialysis bag.

4. The preparation method of claim 3, wherein the oxidoreductase is modified for twice using the ruthenium complex having free amino or carboxyl groups or the osmium complex having free amino groups, wherein the molecular weight cut off in the preliminary modification is 5000 to 50000, and the molecular weight cut off in the secondary modification is 500 to 50000.

5. The preparation method of claim 4, wherein the osmium complex having free amino or carboxyl groups is Os(bpy)₂(3-aminopropyl imidazole)Cl or Os(bpy)₂(4-imidazole butyric acid)Cl, and the ruthenium complex having free amino or carboxyl groups is Ru(bpy)₂(3-aminopropyl imidazole)Cl or Os(bpy)₂(4-imidazole butyric acid)Cl.

6. The preparation method of any one of claims 1-5, wherein performing a cross-linking treatment using a chemical cross-linking agent, comprising the steps of: uniformly mixing the modified oxidoreductase with the chemical cross-linking agent in a buffer solution, reacting for 0.5-5 hours and coating on the surface of the electrode, thereby forming a biosensing membrane.

7. The preparation method of claim 6, wherein after the biosensing membrane formed by the cross-linking treatment is dried, a polyvinyl pyridine and a Nafion mixed alcoholic solution are coated on the surface of the biosensing membrane, thereby forming a biosensing membrane with biocompatibility.

## Patentansprüche

1. Ein Verfahren zur Herstellung einer biosensorischen Membran, das durch die folgenden Schritte gekennzeichnet ist: Elektrochemische Aktivierung und Modifizierung einer Oxidoreduktase unter Verwendung eines Ruthenium-Komplexes mit freien Amino- oder Carboxylgruppen oder eines Osmium-Komplexes mit freien Amino- oder Carboxylgruppen mit anschließender Durchführung einer Vernetzungsbehandlung mittels eines chemischen Vernetzungsmittels und nachfolgender Beschichtung der Oberfläche einer Elektrode, wodurch eine biosensorische Membran gebildet wird, wobei das chemische Vernetzungsmittel Glutaraldehyd oder Polyethylenglykoldiglycidylether ist.

2. Das Herstellungsverfahren nach Anspruch 1, worin die Oxidoreduktase aus einem oder mehreren Stoffen besteht, die aus Glukoseoxidase, Glucosedehydrogenase, Lactatoxidase, Lactatdehydrogenase und Katalase ausgewählt werden.

3. Das Herstellungsverfahren nach Anspruch 1, worin die elektrochemische Aktivierung und Modifizierung der Oxidoreduktase folgende Schritte umfasst: Gleichmäßige Durchmischung der Oxidoreduktase mit dem Ruthenium-Komplex mit freien Amino- oder Carboxylgruppen oder dem Osmium-Komplex mit freien Aminogruppen in einer Pufferlösung, nachfolgendes Hinzufügen von Carbodiimid und N-Hydroxysuccinimid, gleichmäßige Durchmischung und Reaktion bei einer Temperatur von 2-6 °C für 12-48 Stunden und Durchführung der Dialyse in einem Dialysebeutel.

4. Das Herstellungsverfahren nach Anspruch 3, worin die Oxidoreduktase zweimal, unter Verwendung des Ruthenium-Komplexes mit freien Amino- oder Carboxylgruppen oder des Osmium-Komplexes mit freien Aminogruppen modifiziert wird, worin der Molekulargewicht-Grenzwert in der Vormodifikation 5000 bis 50000 beträgt und der Molekulargewicht-Grenzwert in der sekundären Modifikation 500 bis 50000 beträgt.

5. Das Herstellungsverfahren nach Anspruch 4, worin der Osmium-Komplex mit freien Amino- oder Carboxylgruppen Os(bpy)2(3-Aminopropylimidazol)Cl oder Os(bpy)2(4-Imidazol-Buttersäure)Cl bildet und der Ruthenium-Komplex mit freien Amino- oder Carboxylgruppen Ru(bpy)2(3-Aminopropylimidazol)Cl oder Os(bpy)2(4-Imidazol-Buttersäure)Cl bildet.

6. Das Herstellungsverfahren nach einem der Ansprüche 1-5, worin eine Vernetzungsbehandlung unter Verwendung eines chemischen Vernetzungsmittels durchgeführt wird, welche folgende Schritte umfasst: Gleichmäßige Mischung der modifizierten Oxidoreduktase mit dem chemischen Vernetzungsmittel in einer Pufferlösung, Reagieren für 0,5-5 Stunden und Beschichten der Oberfläche der Elektrode zur Bildung einer biosensorischen Membran.

7. Das Herstellungsverfahren nach Anspruch 6, worin eine mit Polyvinylpyridin und Nafion gemischte Alkohollösung auf die Oberfläche der, durch die Vernetzungsbehandlung gebildete biosensorische Membran nach dem Trocknen aufgetragen wird, wodurch eine biosensorische Membran mit Biokompatibilität gebildet wird.

## Revendications

1. Procédé de préparation d'une membrane de biodétection, **caractérisé en ce qu'**il comprend les étapes : de l'activation et de la modification électrochimique d'une oxydoréductase à l'aide d'un complexe de ruthénium ayant des groupes amino ou carboxyle libres ou d'un complexe d'osmium ayant des groupes amino ou carboxyle libres, puis de la réalisation d'un traitement de réticulation à l'aide d'un agent de réticulation chimique, et ensuite du dépôt sur une surface d'une électrode, formant ainsi une membrane de biodétection, dans lequel l'agent de réticulation chimique est du glutaraldéhyde ou de l'éther diglycidylique de polyéthylène glycol.

2. Procédé de préparation selon la revendication 1, dans lequel l'oxydoréductase est une ou plusieurs substances sélectionnées parmi la glucose oxydase, la glucose déshydrogénase, la lactate oxydase, la lactate déshydrogénase et la catalase.

3. Procédé de préparation selon la revendication 1, dans lequel l'activation et la modification électrochimique de l'oxydoréductase, comprend les étapes : du mélange uniforme de l'oxydoréductase avec le complexe de ruthénium ayant des groupes amino ou carboxyle libres ou le complexe d'osmium ayant des groupes amino libres dans une solution tampon, de l'ajout séquentiellement de carbodiimide et de N-hydroxysuccinimide, du mélange uniforme, réagissant à une température de 2-6 °C pendant 12-48 heures, et la réalisation de la dialyse à l'aide d'un sac de dialyse

4. Procédé de préparation selon la revendication 3, dans lequel l'oxydoréductase est modifiée pour deux fois à l'aide du complexe de ruthénium ayant des groupes amino ou carboxy libres ou du complexe d'osmium ayant des groupes amino libres, dans lequel le poids moléculaire coupé dans la modification préliminaire est de 5000 à 50 000, et le poids moléculaire coupé dans la modification secondaire est de 500 à 50 000.

5. Procédé de préparation selon la revendication 4, dans lequel le complexe d'osmium ayant des groupes amino ou carboxyle libres est Os(bpy)2(3-aminopropylimidazole)Cl ou Os(bpy)2(4-imidazole acide butyrique)Cl, et le complexe de ruthénium ayant des groupes amino ou carboxyle libres est Ru(bpy)2(3-aminopropylimidazole)Cl ou Os(bpy)2(acide 4-imidazole acide butyrique)Cl.

6. Procédé de préparation selon l'une quelconque des revendications 1-5, dans lequel la réalisation d'un traitement de réticulation à l'aide d'un agent de réticulation chimique, comprend les étapes : du mélange uniforme de l'oxydoréductase modifiée avec l'agent de réticulation chimique dans une solution tampon , de la réaction pendant 0,5-5 heures et du dépôt sur la surface de l'électrode, formant ainsi une membrane de biodétection.

7. Procédé de préparation selon la revendication 6, dans lequel après que la membrane de biodétection formée par le traitement de réticulation est séchée, une polyvinylpyridine et une solution alcoolique mixte de Nafion sont déposées sur la surface de la membrane de biodétection, formant ainsi une membrane de biodétection à biocompatibilité.
